# EUROPÄISCHE PATENTSCHRIFT

(11) **EP 1 663 946 B1**
(45) Veröffentlichungstag und Bekanntmachung des Hinweises auf die Patenterteilung: **12.12.2007**
(21) Anmeldenummer: 04764997.5
(22) Anmeldetag: 09.09.2004
(51) Int. Cl.: C07C 209/48, C07C 211/27

(54) **VERFAHREN ZUR HERSTELLUNG VON XYLYLENDIAMIN DURCH KONTINUIERLICHE HYDRIERUNG VON FLÜSSIGEM PHTHALODINITRIL**
METHOD FOR THE PRODUCTION OF DIAMINOXYLENE BY CONTINUOUS HYDROGENATION OF LIQUID PHTHALONITRILE
PROCEDE DE PRODUCTION DE XYLYLENEDIAMINE PAR HYDROGENATION CONTINUE DE PHTALODINITRILE LIQUIDE

(30) Priorität: 10.09.2003 DE 10341612; 10.09.2003 DE 10341633; 10.09.2003 DE 10341632; 10.09.2003 DE 10341615; 10.09.2003 DE 10341613; 10.09.2003 DE 10341614; 02.09.2004 DE 102004042954
(43) Veröffentlichungstag der Anmeldung: 07.06.2006
(73) Patentinhaber: BASF Aktiengesellschaft, 67056 Ludwigshafen (DE)
(72) Erfinder: HUGO, Randolf, 67246 Dirmstein (DE); WENZ, Kirsten, 68161 Mannheim (DE); JOURDAN, Sabine, 68163 Mannheim (DE); PREISS, Thomas, 67256 Weisenheim (DE)
(86) Internationale Anmeldenummer: PCT/EP2004/010062
(87) Internationale Veröffentlichungsnummer: WO 2005/026098

(56) Entgegenhaltungen:
- EP-A- 1 193 244
- EP-A- 1 279 661
- DE-B- 1 119 285
- US-A- 4 482 741

## Beschreibung

Die vorliegende Erfindung betrifft ein Verfahren zur Herstellung von Xylylendiamin durch kontinuierliche Hydrierung von flüssigem Phthalodinitril an einem Heterogenkatalysator in Gegenwart von flüssigem Ammoniak in einem Reaktor, bei dem ein Teil des Reaktoraustrags als Umlaufstrom kontinuierlich zum Reaktoreingang zurückgeführt wird (Kreislauffahrweise).

Xylylendiamin (Bis(aminomethyl)benzol) ist ein nützlicher Ausgangsstoff, z.B. für die Synthese von Polyamiden, Epoxyhärtern oder als Zwischenstufe zur Herstellung von Isocyanaten.

Die Bezeichnung "Xylylendiamin" (XDA) umfasst die drei Isomere ortho-Xylylendiamin, meta-Xylylendiamin (MXDA) und para-Xylylendiamin.

Der Begriff "Phthalodinitril" (PDN) umfasst die drei Isomere 1,2-Dicyanbenzol = o-Phthalodinitril, 1,3-Dicyanbenzol = Isophthalodinitril = IPDN und 1,4-Dicyanbenzol = Terephthalodinitril.

Die Phthalodinitrile sind Feststoffe (z.B. schmilzt Isophthalodinitril (IPDN) bei 161°C) und weisen relativ schlechte Löslichkeiten in organischen Lösungsmitteln auf.

Die zweistufige Synthese von Xylylendiamin durch Ammonoxidation von Xylol und anschließender Hydrierung des erhaltenen Phthalodinitrils ist bekannt.

Unumgesetzte Dinitrile lassen sich nur sehr schwer vom XDA destillativ trennen.

US-A-4,482,741 (UOP Inc.) beschreibt die Hydrierung von PDN in Gegenwart von Ammoniak, einem spezifischen Katalysator und XDA als Lösungsmittel.

In MXDA beträgt die Löslichkeit von IPDN bei 70 °C ca. 20 Gew.-%.

EP-A2-1 193 247 und EP-A1-1 279 661 (beide Mitsubishi Gas Chem. Comp.) betreffen ein Verfahren zur Reinigung von Isophthalodinitril (IPDN) bzw. ein Verfahren zur Herstellung von reinem XDA.

EP-A2-1 193 244 (Mitsubishi Gas Chem. Comp.) beschreibt ein Verfahren zur Herstellung von XDA durch Hydrierung von Phthalodinitril, welches in einer vorherigen Stufe durch Ammonoxidation von Xylol synthetisiert wird, wobei das dampfförmige Produkt der Ammonoxidationsstufe direkt mit einem flüssigen organischen Lösungsmittel in Kontakt gebracht wird (Quench) und die erhaltene Quenchlösung oder -suspension der Hydrierung zugeführt wird.

Bevorzugte organische Lösungsmittel sind C₆-C₁₂ aromatische Kohlenwasserstoffe, wie Xylol und Pseudocumol (Spalte 6, Absatz [0027] und [0028]).

US-A-3,069,469 (California Research Corp.) lehrt als Lösungsmittel zur Hydrierung von aromatischen Nitrilen, wie PDN, aromatische Kohlenwasserstoffe, Xylol, Dioxan und aliphatische Alkohole.

DE-A-21 64169 (Mitsubishi Gas Chemical Co., Inc.) beschreibt auf Seite 6, letzter Absatz, die Hydrierung von IPDN zu meta-XDA in Gegenwart eines Ni- und/oder Co-Katalysators in Ammoniak als Lösungsmittel.

GB-A-852,972 (Äquivalent: DE-A-11 19 285) (BASF AG) offenbart die Verwendung von Ammoniak und XDA als Lösungsmittel in der Hydrierung von PDN. Die Herstellung der Edukt-Lösung ausgehend von festem PDN erfolgt in einem Extraschritt in einem separaten Gefäß (vergl. Seite 2, Zeilen 119-120).

JP-A-2003-327563 (Mitsubishi Gas Chem. Co., Inc.) betrifft ein Verfahren zur Festbett-Hydrierung von aromatischen Dinitrilen, die als 1-10 Gew.-%ige Lösungen eingesetzt werden.

Die sechs deutschen Patentanmeldungen mit den Aktenzeichen 10341615.3, 10341632.3, 10341614.5, 10341633.1, 10341612.9 und 10341613.7 (BASF AG) vom 10.09.03 betreffen jeweils Verfahren zur Herstellung von XDA.

Die deutsche Patentanmeldung Nr. 102004042947.2 vom 02.09.04 (BASF AG) betrifft ein Verfahren zur Herstellung von XDA durch kontinuierliche Hydrierung von flüssigem PDN an einem Heterogenkatalysator in Gegenwart von flüssigem Ammoniak in einem Reaktor, wobei mittels einer Mischeinrichtung ein Strom einer Phthalodinitrilschmelze flüssig in einen Strom von flüssigem Ammoniak zugefahren wird und die flüssige Mischung in den Hydrierreaktor gefahren wird.

Bei den unterschiedlichen Verfahren zur Herstellung von Phthalodinitril fällt dieses als Feststoff oder gelöst in einem Lösungsmittel, z.B. Pseudocumol, oder als Schmelze an. Die Handhabung von Feststoffen ist üblicherweise schwierig und umständlich. Die Weiterverarbeitung in einem Lösungsmittel erfordert wegen der geringen Löslichkeit von Phthalodinitril in Lösungsmitteln wie o-Xylol, m-Xylol, p-Xylol, Pseudocumol, Mesitylen, Ethylbenzol oder Methylpyridin sehr große Lösungsmittelmengen, die nach der Hydrierung in der Regel destillativ abgetrennt werden müssen, was - entsprechend den großen Mengenströmen - große Apparate und einen hohen Energieaufwand erfordert.

Alternativ ist eine Extraktion des PDNs mit Wasser mit anschließender Destillation möglich. Auch hier ist der Energieaufwand groß, da das Wasser abdestilliert und das Lösungsmittel - zumindest im Teilstrom - regeneriert werden muss.

Der vorliegenden Erfindung lag die Aufgabe zugrunde, ein verbessertes wirtschaftliches Verfahren zur Herstellung von Xylylendiamin, insbesondere meta-Xylylendiamin, mit hoher Selektivität, Ausbeute und Raum-Zeit-Ausbeute (RZA) aufzufinden, welches bei mit Verfahren des Stands der Technik vergleichbaren Durchsätzen aufgrund verringerter Stoffströme, insbesondere Lösungsmittelströme, inkl. Rückführströme, verkleinerte und/oder weniger Apparate und Maschinen ermöglicht.

Demgemäß wurde ein Verfahren zur Herstellung von Xylylendiamin durch kontinuierliche Hydrierung von flüssigem Phthalodinitril an einem Heterogenkatalysator in Gegenwart von flüssigem Ammoniak in einem Reaktor, bei dem ein Teil des Reaktoraustrags als flüssiger Umlaufstrom kontinuierlich zum Reaktoreingang zurückgeführt wird (Kreislauffahrweise), gefunden, welches dadurch gekennzeichnet ist, dass mittels einer Mischeinrichtung ein Strom einer Phthalodinitrilschmelze flüssig in den Umlaufstrom um den Hydrierreaktor zugefahren wird, wobei der Phthalodinitril-Umsatz im Reaktor bei einfachem Durchgang größer 99 % beträgt, und der Umlaufstrom zu größer 93 Gew.-% aus flüssigem Ammoniak und Xylylendiamin besteht und kein weiteres Lösungsmittel für Phthalodinitril enthält.

Bevorzugt findet das erfindungsgemäße Verfahren Anwendung zur Herstellung von meta-Xylylendiamin (MXDA) durch Hydrierung von Isophthalodinitril (IPDN), welches insbesondere in einer vorherigen Stufe durch Ammonoxidation von meta-Xylol synthetisiert wurde.

Das geschmolzene Phthalodinitril kann beispielsweise aus einem einer Ammonoxidation nachgeschalteten Quench, einer Eindampfstufe oder einer Destillationskolonne kommen, wobei das Phthalodinitril z.B. jeweils als Schmelze über Sumpf dieser thermischen Trennapparate abgetrennt wird, wie z.B. in der deutschen Patentanmeldung Nr. 10341633.1 vom 10.09.03 (BASF AG) beschrieben.

Alternativ kann im erfindungsgemäßen Verfahren auch aufgeschmolzenes, zuvor als Feststoff vorliegendes PDN eingesetzt werden. Das Aufschmelzen kann z.B. mittels eines Extruders erfolgen.

Vorteil der Eindosierung des PDNs als Schmelze in den Umlaufstrom um den Hydrierreaktor ist die recht hohe Verdünnung bei der Eindosierung, schnelle Abkühlung nach Einmischung und somit Reaktionsverhinderung zwischen Nitril und Produkt-Amin. Dadurch wird die Nebenproduktbildung verringert.

Die hohe Verdünnung ist auch während der Reaktion von Vorteil, da die Reaktionswärme so konvektiv (d.h. mit dem erwärmten Reaktoraustrag) abgeführt werden kann. Die Temperaturerhöhung im Reaktor lässt sich so begrenzen. Durch die Einstellung der Reaktorzulauftemperatur und der Größe des Kreislaufstromes lässt sich der Temperaturverlauf im Reaktor beeinflussen. Dabei führt eine niedrigere Reaktortemperatur zu einer weiteren Erhöhung der Selektivität.

Durch die Verdünnung des PDNs mit dem Kreislaufstrom wird im Reaktor eine hohe Ammoniakkonzentration bzgl. PDN erreicht, was sich wiederum günstig auf die Selektivität auswirkt. Dennoch ist nur ein kleiner frisch-NH₃-Strom notwendig und nur eine kleine Ammoniak-Kolonne zur Rückgewinnung und Rückführung des zusammen mit dem Xylylendiamin aus dem Reaktionskreis entnommenen Ammoniaks. Ohne Kreislaufstrom müsste zur Einstellung der gleichen Reaktionsbedingungen mehr Ammoniak zugefahren und danach wieder abdestilliert werden.

Das Zufahren und Lösen der Phthalodinitrilschmelze in der Kreislauflösung (im Umlaufstrom) erfordert eine Mischeinrichtung, bevorzugt eine Mischdüse, welche im einfachsten Fall durch ein Rohrleitungs-T-Stück realisiert werden kann. Bevorzugt weist der Düsenmund eine Verjüngung auf.

Die Ströme werden getrennt zugeführt und im anschließenden Rohr auf Grund der herrschenden Turbulenz vermischt und homogenisiert. Vorteilhaft kann zusätzlich ein statischer Mischer nachgeschaltet werden. Es wird jedoch kein zusätzlicher Apparat, wie etwa ein Rührkessel zum Lösen von (festem oder flüssigem) Phthalodinitril in einem Lösungsmittel benötigt.

Bevorzugt ist die Mischeinrichtung am Ort der Phthalodinitrilzufuhr in den Umlaufstrom auf eine Temperatur im Bereich von 1 bis 40°C, besonders im Bereich von 5 bis 25°C, oberhalb des Schmelzpunktes des eingesetzten Phthalodinitrils beheizt.

Bevorzugt erfolgt die PDN-Zufuhr praktisch bei Reaktordruck. Besonders bevorzugt erfolgt die PDN-Zufuhr derart, dass bei der sich einstellenden Mischtemperatur noch keine Verdampfung einsetzt, sondern die Mischung flüssig bleibt.

Besonders bevorzugt wird mittels einer Mischdüse als Mischeinrichtung das flüssige Phthalodinitril in den Umlaufstrom eingedüst.

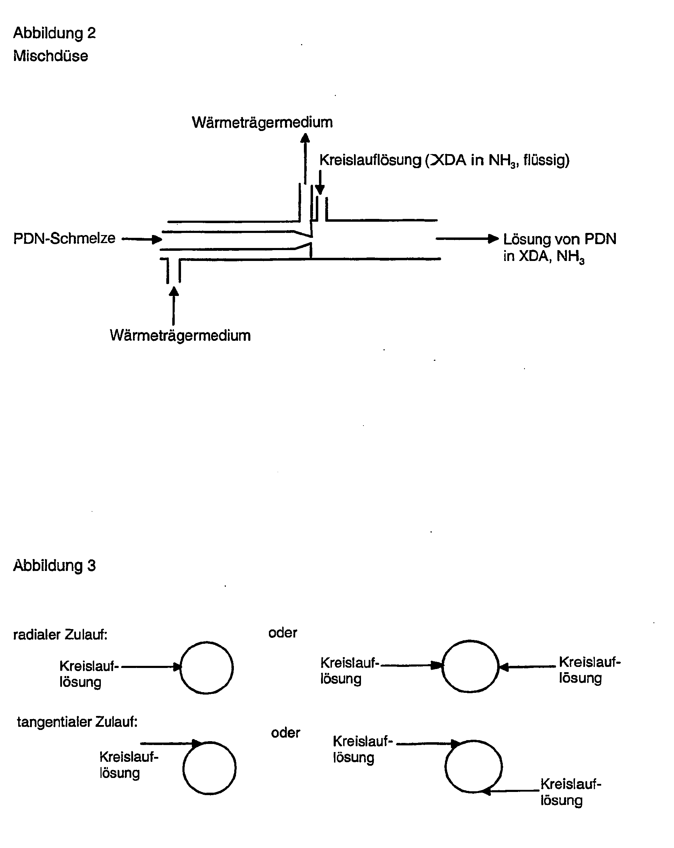

Eine bevorzugte Ausführungsform der Mischdüse ist in Abbildung 2 im Anhang dargestellt. Die Beheizung der Mischdüse kann z.B. mit Dampf, Wärmeträgeröl oder auch elektrisch erfolgen.

Der Zulauf der Kreislauflösung kann über einen oder mehrere radial oder tangential angebrachte Stutzen erfolgen, z.B. wie in der Abbildung 3 gezeigt.

Wichtig ist die lokal hohe Strömungsgeschwindigkeit (hoher Impulsstrom und Turbulenz), so dass eine schnelle Vermischung (Homogenisierung) eintritt. Bei laminarer Strömung reicht der Stoffaustausch zur Homogenisierung nicht aus und die Ströme werden nur unzureichend gemischt (Schlierenbildung).

Geeignete Mischdüsen sind z.B. in Ullmann's Encyclopedia of Industrial Chemistry, 5. Auflage, Vol. B4, Seiten 565 - 569, beschrieben.

Der Phthalodinitril-Umsatz im Hydrierreaktor beträgt bei einfachem Durchgang bevorzugt größer 99,5 %, besonders größer 99,9 %, insbesondere größer 99,95 %, ganz besonders größer 99,97 %. Im Hydrierreaktor wird also durch entsprechende Einstellung der Reaktionsbedingungen (Druck, Temperatur, Molverhältnisse von PDN, NH₃ und H₂, Katalysator, Mengenströme, Verweilzeit im Reaktor) praktisch Vollumsatz gefahren.

Bevorzugt besteht der flüssige Umlaufstrom zu größer 94 Gew.-%, insbesondere größer 95 Gew.-%, ganz besonders größer 96 Gew.-%, aus flüssigem Ammoniak und Xylylendiamin; den Rest bilden Nebenkomponenten.

Nebenkomponenten im flüssigen Umlaufstrom (Kreislaufstrom) können bei der Reaktion gebildete Nebenprodukte sowie gelöste Gase und mit dem Phthalodinitril zugefahrene Nebenkomponenten, jedoch kein weiteres Lösungsmittel, z.B. organisches Lösungsmittel, für Phthalodinitril sein.

Der Umlaufstrom enthält bevorzugt im Bereich von 25 bis 90 Gew.-%, besonders 30 bis 70 Gew.-%, insbesondere 45 bis 60 Gew.-%, flüssigen Ammoniak.

Der Teil des flüssigen Reaktoraustrags, der als Umlaufstrom kontinuierlich zum Reaktoreingang zurückgeführt wird, macht bevorzugt 20 bis 95 Gew.-%, besonders 50 bis 92 Gew.-%, insbesondere 75 bis 90 Gew.-%, des gesamten flüssigen Reaktoraustrags aus.

Das Gewichtsverhältnis von Phthalodinitril-Zulaufstrom zu Umlaufstrom liegt bevorzugt im Bereich von 0,03 bis 1,0, besonders im Bereich von 0,05 bis 0,5, insbesondere im Bereich von 0,07 bis 0,2.

Die Reaktionstemperatur liegt bevorzugt im Bereich von 40 bis 150°C, besonders bevorzugt 60 bis 135°C, insbesondere 70 bis 130°C.

Die Menge des Kreislaufstromes und die Reaktorzulauftemperatur werden so eingestellt, dass die Reaktoraustrittstemperatur den gewünschten Maximalwert (z.B. 130°C) nicht überschreitet, da mit zunehmender Temperatur verstärkt Nebenprodukte gebildet werden. Die Reaktorzulauftemperatur wird so eingestellt (z.B. durch einen zusätzlichen Wärmeübertrager oder, bevorzugt, durch geeignete Einstellung der Temperatur der zu mischenden Ströme), dass die Reaktion hinreichend schnell verläuft und Vollumsatz erreicht wird. Durch Variation des Kreislaufmengenstroms ist es somit möglich, sowohl Eintritts- als auch Austrittstemperatur des Reaktors einzustellen und optimal an die ablaufenden Reaktionen anzupassen und so die XDA-Ausbeute zu optimieren.

Die Hydrierung wird bevorzugt bei einem Absolutdruck im Bereich von 100 bis 300 bar, insbesondere 120 bis 220 bar, ganz besonders 150 bis 200 bar, durchgeführt.

Für die Hydrierung können dem Fachmann bekannte Katalysatoren und Reaktoren (insbesondere Rohrreaktoren oder Rohrbündelreaktoren; Festbett- oder Suspensionsfahrweise) angewendet werden.

Bei der Katalysatorfestbettfahrweise ist sowohl die Sumpf- als auch die Rieselfahrweise möglich. Bevorzugt ist eine Rieselfahrweise.

Bevorzugt wird der Reaktor adiabat betrieben, während die entstehende Reaktionswärme über einen im Umlaufkreis eingebauten Kühler sowie optional mit dem verwendeten Kreisgas abgeführt wird. Dies erhöht zusätzlich die Selektivität der Reaktion durch die weitere Unterdrückung von Nebenprodukten.

Alternativ ist aber auch ein gekühlter Reaktor, beispielsweise ein Rohrbündelreaktor, einsetzbar.

Bevorzugt sind Katalysatoren, die Kobalt und/oder Nickel und/oder Eisen, als Vollkatalysator oder auf einem inerten Träger, enthalten.

Besonders bevorzugt wird die Hydrierung an einem Mangan-dotierten Cobalt-Vollkatalysator durchgeführt.

Geeignete Katalysatoren sind beispielsweise Raney-Nickel, Raney-Cobalt, Co-Vollkontakt, Titan-dotiertes Cobalt auf Träger (JP-A-2002 205980), Ni auf SiO₂-Träger (WO-A-2000/046179), Co/Ti/Pd auf SiO₂-Träger (CN-A-1 285 343, CN-A-1 285 236) und Nickel und/oder Cobalt auf Zirkoniumdioxid-Träger (EP-A1-1 262 232).

Beispiele für weitere geeignete Katalysatoren finden sich z.B. in den Anmeldungen GB-A-852,972 (Äquivalent: DE-A-11 19 285) (BASF AG), DE-A-12 59 899 (BASF AG) und den US Patenten Nr. 3,069,469 (California Research Corp.) und 4,482,741 (UOP Inc.).

Besonders bevorzugte Katalysatoren sind die in EP-A1-742 045 (BASF AG) offenbarten Cobalt-Vollkontakte, dotiert mit Mn, P, und Alkalimetall (Li, Na, K, Rb, Cs).

Die katalytisch aktive Masse dieser Katalysatoren besteht vor der Reduktion mit Wasserstoff aus 55 bis 98 Gew.-%, insbesondere 75 bis 95 Gew.-%, Cobalt, 0,2 bis 15 Gew.-% Phosphor, 0,2 bis 15 Gew.-% Mangan und 0,05 bis 5 Gew.-% Alkalimetall, insbesondere Natrium, jeweils berechnet als Oxid.

Weitere geeignete Katalysatoren sind die in EP-A-963 975 (BASF AG) offenbarten Katalysatoren, deren katalytisch aktive Masse vor der Behandlung mit Wasserstoff
22 bis 40 Gew.-% ZrO₂,
1 bis 30 Gew.-% sauerstoffhaltige Verbindungen des Kupfers, berechnet als CuO,
15 bis 50 Gew.-% sauerstoffhaltige Verbindungen des Nickels, berechnet als NiO, wobei das molare Ni : Cu-Verhältnis größer 1 ist,
15 bis 50 Gew.-% sauerstoffhaltige Verbindungen des Kobalts, berechnet als CoO,
0 bis 10 Gew.-% sauerstoffhaltige Verbindungen des Aluminiums und/oder Mangans, berechnet als Al₂O₃ bzw. MnO₂,
und keine sauerstoffhaltigen Verbindungen des Molybdäns enthält,
beispielsweise der in loc. cit., Seite 17, offenbarte Katalysator A mit der Zusammensetzung 33 Gew.-% Zr, berechnet als ZrO₂, 28 Gew.-% Ni, berechnet als NiO, 11 Gew.-% Cu, berechnet als CuO und 28 Gew.-% Co, berechnet als CoO,
die
in EP-A-696 572 (BASF AG) offenbarten Katalysatoren, deren katalytisch aktive Masse vor der Reduktion mit Wasserstoff 20 bis 85 Gew.-% ZrO₂, 1 bis 30 Gew.-% sauerstoffhaltige Verbindungen des Kupfers, berechnet als CuO, 30 bis 70 Gew.-% sauerstoffhaltige Verbindungen des Nickels, berechnet als NiO, 0,1 bis 5 Gew.-% sauerstoffhaltige Verbindungen des Molybdäns, berechnet als MoO₃, und 0 bis 10 Gew.-% sauerstoffhaltige Verbindungen des Aluminiums und/oder Mangans, berechnet als Al₂O₃ bzw. MnO₂, enthält, beispielsweise der in loc. cit., Seite 8, offenbarte Katalysator mit der Zusammensetzung 31,5 Gew.-% ZrO₂, 50 Gew.-% NiO, 17 Gew.-% CuO und 1,5 Gew.-% MoO₃,
und die in WO-A-99/44984 (BASF AG) beschriebenen Katalysatoren enthaltend (a) Eisen oder eine Verbindung auf der Basis von Eisen oder deren Gemische, (b) von 0,001 bis 0,3 Gew.-% bezogen auf (a) eines Promoters auf der Basis von 2, 3, 4 oder 5 Elementen ausgewählt aus der Gruppe Al, Si, Zr, Ti, V, (c) von 0 bis 0,3 Gew.-% bezogen auf (a) einer Verbindung auf der Basis eines Alkali- und/oder Erdalkalimetalls, sowie (d) von 0,001 bis 1 Gew.-% bezogen auf (a) Mangan.

Das erfindungsgemäße Verfahren lässt sich z.B. wie folgt ausführen:

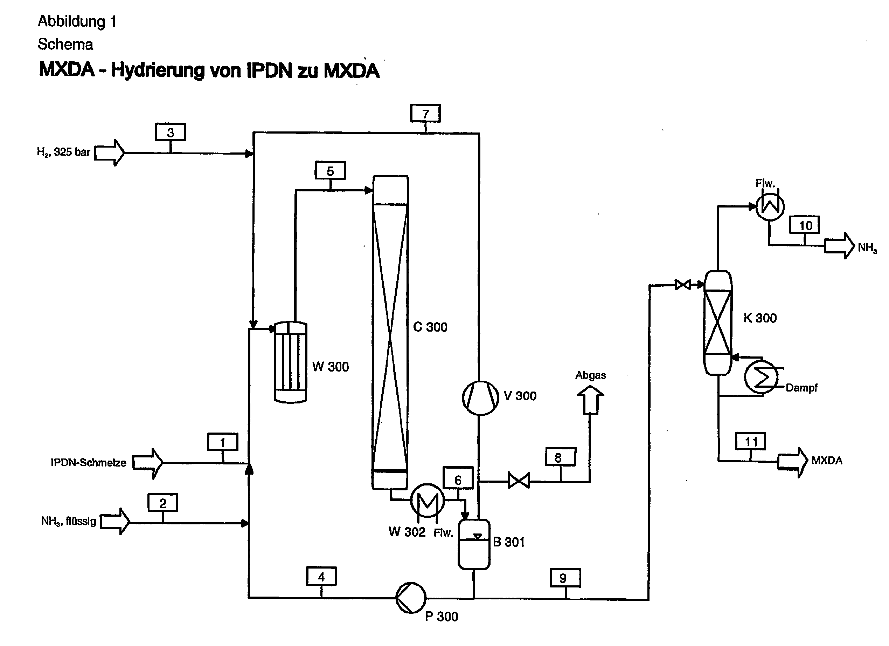

In Abbildung 1 ist eine mögliche Anordnung des Hydrierreaktors mit Umlaufkreis und Wärmeübertragern dargestellt. Die Phthalodinitrilschmelze wird als Strom [1] der Hydrierstufe zugeführt und mit dem Umlaufstrom [4] gemischt. Ammoniak [2] wird in flüssiger Form zugemischt. Dies kann entweder vor dem Mischpunkt mit der Phthalodinitrilschmelze (wie in Abb. 1 gezeigt) oder dahinter geschehen. Wasserstoff und gegebenenfalls Kreisgas werden zugefahren und mittels eines optionalen Wärmeübertragers auf die gewünschte Reaktorzulauftemperatur erwärmt. Gas und Flüssigkeit können auch getrennt dem Reaktor zugefahren werden. Bevorzugt wird die Temperatur der zu mischenden Ströme mittels Wärmeübertragern so eingestellt, dass nach der Vermischung kein Wärmeübertrager mehr erforderlich ist. Gase und Flüssigkeit werden bevorzugt getrennt dem Hydrierreaktor zugeführt. Im Reaktor erfolgt die Hydrierung praktisch quantitativ, so dass im Reaktionsaustrag praktisch kein Phthalodinitril mehr vorhanden ist. Der Reaktionsaustrag kann dann abgekühlt werden und Gas und Flüssigkeit werden unter Druck in einem Hochdruckabscheider getrennt. Die Flüssigkeit wird teilweise ohne Aufarbeitung im Kreis gefahren (Strom [4]) und teilweise der Aufarbeitung (Strom [9]) zugeführt. Ein Teil des Gases wird ausgeschleust, um die Aufpegelung von Inerten (CO, N₂, CH₄, Edelgase, etc.) zu vermeiden. Der größte Teil des Gases wird über einen Verdichter zum Reaktoreingang zurückgeführt. Bei nicht zu hohem Druckverlust im Reaktor kann hierzu bevorzugt auch eine Ejektorstrahldüse ("Wasserstrahlpumpe") verwendet werden. Insgesamt kann die Kreisgasmenge in weiten Bereichen variiert werden, etwa vom mehrfachen der Frischgasmenge bis hin zu Null (Fahrweise ohne Kreisgas). Die Kreisgasfahrweise ist günstig, um den Reaktor für einen guten Stoffübergang ausreichend gasseitig zu belasten und um für Inertgase einen hinreichenden Schleppstrom zur Verfügung zu stellen. Zusätzlich kann ein Teil der Reaktionswärme mit dem Gasstrom abgeführt werden. Mit zunehmender Temperatur verdampft eine zunehmende Menge an Ammoniak, was den kühlenden Effekt des Kreisgases noch verstärkt. Der Reaktionsaustrag (Strom [9]) wird dann zunächst einer Druckdestillation zugeführt, in der flüssiger Ammoniak über Kopf (Strom [10]) und weitgehend ammoniakfreies, rohes Xylylendiamin über Sumpf (Strom [11]) gewonnen werden, wobei der Ammoniak in kondensierter Form wieder der Hydrierstufe zugeführt werden kann. Das rohe Xylylendiamin wird z.B. durch Destillation weiter gereinigt.

Im erfindungsgemäßen Verfahren kann das Gewichtsverhältnis der Frischzuläufe von Dinitril und Ammoniak (z.B. gem. Abbildung 1 das Verhältnis von Strom [1] zu Strom [2]) um so größer gewählt werden, je größer der Kreislaufstrom ist. Bevorzugt beträgt das Gewichtsverhältnis von Dinitril zu Ammoniak 1 : 0,5 bis 1:10, vorzugsweise 1 : 0,6 bis 1 : 5, besonders bevorzugt 1 : 0,9 bis 1 : 3,5.

### Isolierung des XDAs:

Nach der Hydrierung wird der eingesetzte Ammoniak abgetrennt, z.B. abdestilliert.

Bevorzugt erfolgt eine Reinigung des Xylylendiamins durch Abdestillation leichtersiedender Nebenprodukte (bei gleichem Druck) über Kopf und destillativer Abtrennung von schwerersiedenden Verunreinigungen über Sumpf.

Besonders bevorzugt ist die Fahrweise, in der man nach der Hydrierung den Ammoniak sowie gegebenenfalls leichtsiedende Nebenprodukte über Kopf abdestilliert und danach schwerersiedende Verunreinigungen vom Xylylendiamin destillativ über Sumpf abtrennt.

In einer besonderen Ausführungsform kann die Abtrennung leichter- und schwerersiedender Nebenprodukte auch in einer Seitenabzugs- oder Trennwandkolonne erfolgen, wobei reines Xylylendiamin über einen flüssigen oder gasförmigen Seitenabzug gewonnen wird.

Je nach gewünschter Reinheit wird das Produkt (XDA) zusätzlich mit einem organischen Lösungsmittel, bevorzugt einem aliphatischen Kohlenwasserstoff, insbesondere einem cycloaliphatischen Kohlenwasserstoff, ganz besonders Cyclohexan oder Methylcyclohexan, extrahiert.

Diese Reinigung durch Extraktion kann z.B. gemäß DE-A-1 074 592 (BASF AG) erfolgen.

### Beispiel

90 g/h geschmolzenes IPDN (kommerzielles, geschupptes IPDN, welches durch Erhitzen auf ca. 170°C aufgeschmolzen wurde) wurde mittels eines Rohrleitungs-T-Stücks in einen Kreislaufstrom (ca. 1000 g/h) bestehend aus dem flüssigen Rückführstrom des Reaktoraustrags sowie 90 g/h frisch-Ammoniak gefahren. Die erhaltene Reaktionsmischung wurde kontinuierlich in einen Rohreaktor an einem Kobalt-Vollkontakt bei 90°C und 200 bar hydriert. Der abgezogene Teil des Reaktoraustrags wurde in einer Ammoniakkolonne vom Großteil der Ammoniakmenge befreit und über GC-untersucht. Bei Vollumsatz des eingesetzen IPDN (d.h. Umsatz größer 99,95 %; per GC kein Edukt mehr nachweisbar) lag die Selektivität bei 93 %.

In anschließenden Destillationsschritten wurden zuerst Rest-Ammoniak und leichtsiedende Nebenkomponenten abgetrennt. Nach Abtrennung der hochsiedenden Verunreinigungen über Sumpf wurde MXDA als Kopfprodukt einer Destillationskolonne in einer Reinheit von mehr als 99,9 Gew.-% erhalten.

## Patentansprüche

1. Verfahren zur Herstellung von Xylylendiamin durch kontinuierliche Hydrierung von flüssigem Phthalodinitril an einem Heterogenkatalysator in Gegenwart von flüssigem Ammoniak in einem Reaktor, bei dem ein Teil des Reaktoraustrags als flüssiger Umlaufstrom kontinuierlich zum Reaktoreingang zurückgeführt wird (Kreislauffahrweise), **dadurch gekennzeichnet, dass** mittels einer Mischeinrichtung ein Strom einer Phthalodinitrilschmelze flüssig in den Umlaufstrom um den Hydrierreaktor zugefahren wird, wobei der Phthalodinitril-Umsatz im Reaktor bei einfachem Durchgang größer 99 % beträgt, und der Umlaufstrom zu größer 93 Gew.-% aus flüssigem Ammoniak und Xylylendiamin besteht und kein weiteres Lösungsmittel für Phthalodinitril enthält.

2. Verfahren nach Anspruch 1 zur Herstellung von meta-Xylylendiamin durch Hydrierung von Isophthalodinitril.

3. Verfahren nach den Ansprüchen 1 oder 2, **dadurch gekennzeichnet, dass** die Mischeinrichtung am Ort der Phthalodinitrilzufuhr in den Umlaufstrom auf eine Temperatur im Bereich von 1 bis 40°C oberhalb des Schmelzpunktes des eingesetzten Phthalodinitrils beheizt ist.

4. Verfahren nach einem der vorhergehenden Ansprüche, **dadurch gekennzeichnet, dass** mittels einer Mischdüse als Mischeinrichtung das flüssige Phthalodinitril in den Umlaufstrom eingedüst wird.

5. Verfahren nach einem der vorhergehenden Ansprüche, **dadurch gekennzeichnet, dass** der Phthalodinitril-Umsatz im Hydrierreaktor bei einfachem Durchgang größer 99,5 % beträgt.

6. Verfahren nach einem der Ansprüche 1 bis 4, **dadurch gekennzeichnet, dass** der Phthalodinitril-Umsatz im Hydrierreaktor bei einfachem Durchgang größer 99,9 % beträgt.

7. Verfahren nach einem der vorhergehenden Ansprüche, **dadurch gekennzeichnet, dass** der Umlaufstrom zu größer 94 Gew.-% aus flüssigem Ammoniak und Xylylendiamin besteht.

8. Verfahren nach einem der vorhergehenden Ansprüche, **dadurch gekennzeichnet, dass** der Umlaufstrom im Bereich von 25 bis 90 Gew.-% flüssigen Ammoniak enthält.

9. Verfahren nach einem der vorhergehenden Ansprüche, **dadurch gekennzeichnet, dass** der Teil des flüssigen Reaktoraustrags, der als Umlaufstrom kontinuierlich zum Reaktoreingang zurückgeführt wird, 20 bis 95 Gew.-% des gesamten flüssigen Reaktoraustrags ausmacht.

10. Verfahren nach einem der vorhergehenden Ansprüche, **dadurch gekennzeichnet, dass** das Gewichtsverhältnis von Phthalodinitril-Zulaufstrom zu Umlaufstrom im Bereich von 0,03 bis 1,0 liegt.

11. Verfahren nach einem der vorhergehenden Ansprüche, **dadurch gekennzeichnet, dass** die Hydrierung bei einer Temperatur im Bereich von 40 bis 150°C durchgeführt wird.

12. Verfahren nach einem der vorhergehenden Ansprüche, **dadurch gekennzeichnet, dass** die Hydrierung bei einem Absolutdruck im Bereich von 100 bis 300 bar durchgeführt wird.

13. Verfahren nach einem der vorhergehenden Ansprüche, **dadurch gekennzeichnet, dass** die Hydrierung an einem Katalysator enthaltend Ni, Co und/oder Fe, als Vollkatalysator oder auf einem inerten Träger, durchgeführt wird.

14. Verfahren nach einem der vorhergehenden Ansprüche, **dadurch gekennzeichnet, dass** die Hydrierung an einem Mangan-dotierten Cobalt-Vollkatalysator durchgeführt wird.

15. Verfahren nach einem der vorhergehenden Ansprüche, **dadurch gekennzeichnet, dass** der Katalysator in einem Rohrreaktor oder Rohrbündelreaktor als Festbett angeordnet ist.

16. Verfahren nach dem vorhergehenden Anspruch, **dadurch gekennzeichnet, dass** der Reaktor in Rieselfahrweise betrieben wird.

17. Verfahren nach einem der vorhergehenden Ansprüche, **dadurch gekennzeichnet, dass** der Reaktor adiabat betrieben wird.

18. Verfahren nach einem der vorhergehenden Ansprüche, **dadurch gekennzeichnet, dass** dem Umlaufstrom in einem Kühler Wärme entzogen wird.

19. Verfahren nach einem der vorhergehenden Ansprüche, **dadurch gekennzeichnet, dass** nach der Hydrierung eine Reinigung des Xylylendiamins durch Abdestillation des Ammoniaks sowie gegebenenfalls leichtersiedender Nebenprodukte über Kopf und destillativer Abtrennung von schwerersiedenden Verunreinigungen über Sumpf erfolgt.

20. Verfahren nach dem vorhergehenden Anspruch, **dadurch gekennzeichnet, dass** das Xylylendiamin nach der Destillation zur weiteren Reinigung mit einem organischem Lösungsmittel extrahiert wird.

21. Verfahren nach dem vorhergehenden Anspruch, **dadurch gekennzeichnet, dass** man zur Extraktion Cyclohexan oder Methylcyclohexan verwendet.

## Claims

1. A process for preparing xylylenediamine by continuously hydrogenating liquid phthalonitrile over a heterogeneous catalyst in the presence of liquid ammonia in a reactor, in which a portion of the reactor effluent is recycled as a liquid circulation stream continuously to the reactor inlet (circulation mode), which comprises conducting a stream of a phthalonitrile melt in liquid form by means of a mixer unit into the circulation stream around the hydrogenation reactor, the phthalonitrile conversion in the reactor on single pass being greater than 99%, and the circulation stream consisting to an extent of greater than 93% by weight of liquid ammonia and xylylenediamine and not comprising any further solvent for phthalonitrile.

2. The process according to claim 1 for preparing meta-xylylenediamine by hydrogenating isophthalonitrile.

3. The process according to claims 1 or 2, wherein the mixer unit is heated at the point of the phthalonitrile supply into the circulation stream to a temperature in the range from 1 to 40°C above the melting point of the phthalonitrile used.

4. The process according to any of the preceding claims, wherein the liquid phthalonitrile is sprayed into the circulation stream by means of a mixer nozzle as the mixer unit.

5. The process according to any of the preceding claims, wherein the phthalonitrile conversion in the hydrogenation reactor on single pass is greater than 99.5%.

6. The process according to any of claims 1 to 4, wherein the phthalonitrile conversion in the hydrogenation reactor on single pass is greater than 99.9%.

7. The process according to any of the preceding claims, wherein the circulation stream consists to an extent of greater than 94% by weight of liquid ammonia and xylylenediamine.

8. The process according to any of the preceding claims, wherein the circulation stream contains in the range from 25 to 90% by weight of liquid ammonia.

9. The process according to any of the preceding claims, wherein the portion of the liquid reactor effluent which is recycled as the circulation stream continuously to the reactor inlet makes up from 20 to 95% by weight of the overall liquid reactor effluent.

10. The process according to any of the preceding claims, wherein the weight ratio of phthalonitrile feed stream to circulation stream is in the range from 0.03 to 1.0.

11. The process according to any of the preceding claims, wherein the hydrogenation is carried out at a temperature in the range from 40 to 150°C.

12. The process according to any of the preceding claims, wherein the hydrogenation is carried out at an absolute pressure in the range from 100 to 300 bar.

13. The process according to any of the preceding claims, wherein the hydrogenation is carried out over a catalyst comprising Ni, Co and/or Fe, as an unsupported catalyst or on an inert support.

14. The process according to any of the preceding claims, wherein the hydrogenation is carried out over a manganese-doped unsupported cobalt catalyst.

15. The process according to any of the preceding claims, wherein the catalyst is disposed as a fixed bed in a tubular reactor or tube bundle reactor.

16. The process according to the preceding claim, wherein the reactor is operated in trickle mode.

17. The process according to any of the preceding claims, wherein the reactor is operated adiabatically.

18. The process according to any of the preceding claims, wherein heat is withdrawn from the circulation stream in a cooler.

19. The process according to any of the preceding claims, wherein the xylylenediamine is purified after the hydrogenation by distilling off the ammonia and also any relatively low-boiling by-products overhead and distillatively removing relatively high-boiling impurities via the bottom.

20. The process according to the preceding claim, wherein the xylylenediamine is extracted after the distillation with an organic solvent for further purification.

21. The process according to the preceding claim, wherein cyclohexane or methylcyclohexane are used for the extraction.

## Revendications

1. Procédé de fabrication de xylylènediamine par hydrogénation en continu de phtalodinitrile liquide sur un catalyseur hétérogène en présence d'ammoniac liquide dans un réacteur, dans lequel une partie de l'extrait de réacteur est renvoyée en continu à l'entrée du réacteur sous la forme d'un courant de circulation (mode opératoire en circuit), **caractérisé en ce que** l'on délivre, au moyen d'un dispositif de mélange, un courant d'une masse fondue de phtalodinitrile sous forme liquide au courant de circulation autour du réacteur d'hydrogénation, le taux de conversion du phtalodinitrile étant supérieur à 99 % dans le réacteur dans le cas d'un passage unique, et le courant de circulation étant constitué à plus de 93 % en poids d'ammoniac liquide et de xylylènediamine et ne contenant aucun autre solvant pour le phtalodinitrile.

2. Procédé selon la revendication 1 pour la fabrication de méta-xylylènediamine par hydrogénation d'isophtalodinitrile.

3. Procédé selon les revendications 1 ou 2, **caractérisé en ce que** le dispositif de mélange est chauffé à l'endroit où s'effectue l'alimentation en phtalodinitrile dans le courant de circulation, à une température dans la plage de 1 à 40 °C au-dessus du point de fusion du phtalodinitrile utilisé.

4. Procédé selon l'une quelconque des revendications précédentes, **caractérisé en ce que** le phtalodinitrile liquide est injecté dans le courant de circulation au moyen d'une tuyère de mélange comme dispositif de mélange.

5. Procédé selon l'une quelconque des revendications précédentes, **caractérisé en ce que** le taux de conversion du phtalodinitrile dans le réacteur d'hydrogénation est supérieur à 99,5 % dans le cas d'un passage unique.

6. Procédé selon l'une quelconque des revendications 1 à 4, **caractérisé en ce que** le taux de conversion du phtalodinitrile dans le réacteur d'hydrogénation est supérieur à 99,9 % dans le cas d'un passage unique.

7. Procédé selon l'une quelconque des revendications précédentes, **caractérisé en ce que** le courant de circulation est constitué à plus de 94 % en poids d'ammoniac liquide et de xylylènediamine.

8. Procédé selon l'une quelconque des revendications précédentes, **caractérisé en ce que** le courant de circulation contient de l'ammoniac liquide dans la plage de 25 à 90 % en poids.

9. Procédé selon l'une quelconque des revendications précédentes, **caractérisé en ce que** la partie de l'extrait de réacteur liquide, qui est continuellement renvoyée à l'entrée du réacteur comme courant de circulation, se compose de 20 à 95 % en poids de l'ensemble de l'extrait de réacteur liquide.

10. Procédé selon l'une quelconque des revendications précédentes, **caractérisé en ce que** le rapport pondéral du courant d'alimentation de phtalodinitrile au courant de circulation se situe dans la plage de 0,03 à 1,0.

11. Procédé selon l'une quelconque des revendications précédentes, **caractérisé en ce que** l'on réalise l'hydrogénation à une température dans la plage de 40 à 150 °C.

12. Procédé selon l'une quelconque des revendications précédentes, **caractérisé en ce que** l'on réalise l'hydrogénation à une pression absolue dans la plage de 100 à 300 bars.

13. Procédé selon l'une quelconque des revendications précédentes, **caractérisé en ce que** l'hydrogénation est effectuée sur un catalyseur contenant du Ni, du Co et/ou du Fe, sous la forme de catalyseur plein ou sous une forme déposée sur un support inerte.

14. Procédé selon l'une quelconque des revendications précédentes, **caractérisé en ce que** l'hydrogénation est effectuée sur un catalyseur plein de cobalt dopé avec du manganèse.

15. Procédé selon l'une quelconque des revendications précédentes, **caractérisé en ce que** le catalyseur est aménagé dans un réacteur tubulaire ou dans un réacteur à faisceaux tubulaires sous la forme d'un lit fixe.

16. Procédé selon la revendication précédente, **caractérisé en ce que** le réacteur est exploité selon le mode opératoire de ruissellement.

17. Procédé selon l'une quelconque des revendications précédentes, **caractérisé en ce que** le réacteur est exploité en mode adiabatique.

18. Procédé selon l'une quelconque des revendications précédentes, **caractérisé en ce que** l'on dissipe la chaleur du courant de circulation dans un dispositif de refroidissement.

19. Procédé selon l'une quelconque des revendications précédentes, **caractérisé en ce que** l'on effectue, après l'hydrogénation, une purification de la xylylènediamine en éliminant par distillation l'ammoniac et, éventuellement, les sous-produits à point d'ébullition plus faible en tête de colonne, et en séparant par distillation les impuretés à point d'ébullition plus élevé en bas de colonne.

20. Procédé selon la revendication précédente, **caractérisé en ce que** la xylylènediamine est extraite après la distillation avec un solvant organique pour une purification supplémentaire.

21. Procédé selon la revendication précédente, **caractérisé en ce que** l'on utilise du cyclohexane ou du méthylcyclohexane pour l'extraction.
